## Europäisches Patentamt

**European Patent Office**

**Office européen des brevets**

(11) Publication number: **0 448 360 A1**

(12) # EUROPEAN PATENT APPLICATION

(21) Application number: 91302380.0

(22) Date of filing: 19.03.91

(51) Int. Cl.$^5$: **G01N 33/00**

(30) Priority: 21.03.90 US 496707

(43) Date of publication of application:
25.09.91 Bulletin 91/39

(84) Designated Contracting States:
BE CH DE ES FR GB IT LI NL

(71) Applicant: WESTINGHOUSE ELECTRIC
CORPORATION
Westinghouse Building Gateway Center
Pittsburgh Pennsylvania 15222 (US)

(72) Inventor: Iacovino, John M., Jr.
5210 Mamont Road
Murrysville, PA 15668 (US)
Inventor: Liparulo, Nicholas James
12420 Longview Drive
North Huntingdon, PA 15642 (US)

(74) Representative: van Berlyn, Ronald Gilbert
23, Centre Heights
London, NW3 6JG (GB)

(54) **Chemical monitoring system.**

(57) A data processing unit is linked to sensing hardware for sensing chemical constituents and chemical concentrations, to a meteorological data source and to a data input device. The processing unit may be controlled by well-known software or devices designed to sample data provided by the sensing hardware at predetermined times or at predetermined frequencies. The processing unit may employ meteorological data, such as wind speed and wind direction data, received from a real-time meteorological data source (e.g., a local weather station, or a portable meteorological station) along with sensed chemical concentration and constituent data received from real-time chemical sensing hardware (e.g., a chromatograph) to develop a model of the probable distribution (extent of spreading or plume) of airborne chemicals and/or the chemical source and its location. The model may be developed by the processing unit in conjunction with well-known chemical dispersion modelling software. However, due to the interconnection of the processing unit with real-time sensing and data source hardware, the resultant dispersion model can accurately correspond with the instantaneous (real-time) dispersion of airborne chemicals.

EP 0 448 360 A1

FIG.1

## CHEMICAL MONITORING SYSTEM

The present invention relates to a system for monitoring and analyzing airborne chemical constituents and chemical concentrations to provide a model of the expected chemical dispersion.

Various hardware systems for sensing chemical constituents and chemical concentrations (e.g., chromatograph devices) are known in the art. Many conventional sensing systems tend to be rather large and bulky and, as a result, are typically used in closed environments in which the systems remain stationary, for example within the walls of a factory or production plant.

Developments in the chromatography field have lead to smaller, more compact sensing hardware systems. The smaller systems typically employ silicon micromachine technology, wherein much of the sensor hardware is fabricated on a relatively small silicon wafer. An example of such sensing hardware is the M200 microsensor gas analyzer produced by Microsensor Technology, Inc. These relatively compact systems have been provided in portable housings which allow the systems to be readily moved to, and selectively placed in, the environment in which sensing operations are desired.

Systems for monitoring airborne chemical constituents and constituent concentrations are used in a variety of contexts. For example, chemical production plants (as well as other types of facilities which employ or produce chemicals or hazardous substances) typically include airborne chemical sensing systems in or near populated environments (e.g., employee work areas).

Monitoring of airborne chemicals in or near the work place is highly desirable for safety and health reasons and, in certain contexts, may be required by Federal or State regulations. In many situations, it may also be desirable to monitor airborne chemicals in the environment outside of facilities in which hazardous chemicals are produced or stored. For example, in the event of a fire, earthquake, or other disaster, it would be desirable to monitor airborne chemicals in the environment outside of such facilities so that rescue or containment personnel can be forewarned of the type, amount and location of chemicals which may be present within or near the facilities.

In many situations, it would further be desirable to determine the extent to which airborne chemicals have or will spread in an environment within or outside of the facilities (e.g., to determine the actual or probable chemical plume). However, to determine the actual extent of dispersion of airborne chemicals in a large environment would normally have required an extensive array of sensors disposed throughout the environment. Such an extensive array of sensors would not only be extremely expensive, but may also be undesirable for other reasons, such as aesthetics or space considerations.

It is an object of the present invention to provide a monitoring system for monitoring airborne chemicals in an environment and for determining an accurate model of the expected extent to which airborne chemicals have or will spread in the environment.

These and other objects are accomplished, according to an embodiment of the present invention, by linking a data processing unit to sensing hardware for sensing chemical constituents and chemical concentrations, to a meteorological data source and to a data input device. The processing unit may be controlled by well-known software or devices designed to sample data provided by the sensing hardware at predetermined times or at predetermined frequencies.

The processing unit may employ meteorological data, such as wind speed and wind direction data, received from a real-time meteorological data source (e.g., a local weather station, or a portable meteorological station) along with sensed chemical concentration and constituent data received from real-time chemical sensing hardware (e.g., a chromatograph) to develop a model of the probable distribution (extent of spreading or plume) of airborne chemicals. The model may be developed by the processing unit in conjunction with well-known chemical dispersion modelling software. However, due to the interconnection of the processing unit with real-time sensing and data source hardware, the resultant dispersion model can accurately correspond with the instantaneous (real-time) dispersion of airborne chemicals.

As a result, the extent to which airborne chemicals have or will spread in an environment can be predicted and modeled with a relatively high degree of accuracy. Additionally, the source and the location of the source of the sensed chemicals may be calculated and modeled with a relatively high degree of accuracy based on the actual chemical concentrations measured in the environment. Corrections of inaccuracies in, or alterations to, the model can be made with the input device by entering data corresponding to known parameters or known factors (e.g., a known amount of chemicals which existed or remain in the facility being monitored).

Moreover, the data link connecting the processing unit and the sensing hardware can be designed to allow the sensing hardware to be located remotely from the processing unit. In this regard, the sensing hardware may be positioned in an environment in which hazardous chemicals may exist, while the processing unit may be located in a control facility at a safe distance from the hazardous chemicals.

It is yet another object of the present invention to provide a monitoring system which can employ real-time data, such as chromatographic data, meteorological data and specific site data, from various sources in determining the real-time probable extent of spreading of the airborne chemicals and/or the chemical source and its location.

It is an advantage of the present invention in that it provides a monitoring system which is relatively economical to produce, install and use.

The detailed description of the invention will be made with reference to the accompanying drawings, wherein like numerals designate corresponding parts in the several Figures.

Figure 1 is a schematic diagram of a monitoring system according to an embodiment of the present invention.

Figure 2 is a block diagram of the embodiment shown in Figure 1.

The following detailed description is of the best presently contemplated mode of carrying out the invention. This description is not to be taken in a limiting sense, but is made merely for the purpose of illustrating the general principles of the invention. The scope of the invention is best defined by the appended claims.

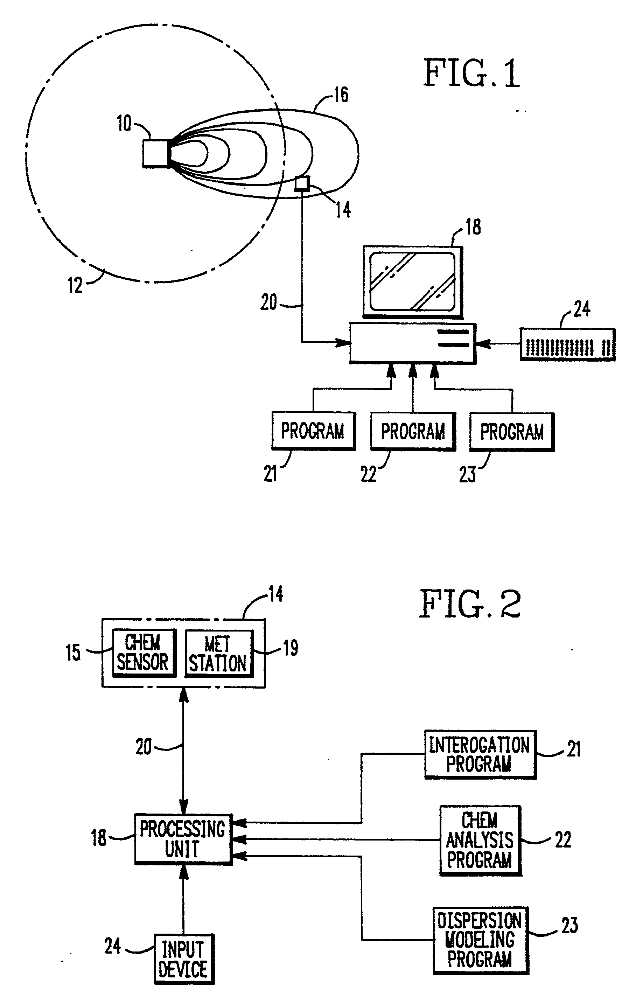

Figures 1 and 2 are schematic and block diagrams, respectively, representing an embodiment of the present invention. In Figure 1, a facility 10 is shown as being located within a site boundary 12. Facility 10 may be a chemical production plant, a facility which employs or produces hazardous chemicals, a hazardous waste site, or the like. As discussed above, it may be desirable, if not required by local or Federal regulations, to monitor the chemical constituents and chemical concentrations of airborne chemicals in and around facility 10 and site boundary 12.

In Figure 1, a sensing device 14 is located outside of site boundary 12 for purposes of illustrating an embodiment of the present invention. It will be recognized, however, that sensing device 14 may be provided within site boundary 12, within facility 10, or at a greater or smaller distance from site boundary 12 than that at which it is shown in Figure 1. Additionally, it will be recognized that several sensing devices may be located at various locations as described above.

Sensing device 14 comprises a chemical constituent and chemical concentration sensor 15, preferably a portable real-time chromatograph device which employs silicon micromachining technology, such as the M200 microsensor gas analyzer produced by Microsensor Technology, Inc. Such portable devices are preferred because they can be readily moved to, and installed at, various locations in and around facility 10 and site 12. It is also preferable that the chemical sensor 15 of sensing device 14 be a real-time device, so as to provide real-time chemical concentration and constituent information. However, it will be recognized that other suitable chemical constituent and chemical concentration sensing devices may be employed.

As shown in Figure 1, a chemical plume 16 is emanating from facility 10. Chemical plume 16 may be a result of normal operations within facility 10 or may be a result of an inadvertent chemical release due to a disaster (fire, earthquake, etc.), equipment malfunction, or the like. In either case, it may be desirable to monitor the chemical constituents and chemical concentrations within plume 16 and the environment about facility 10. Monitoring of such chemical constituents and chemical concentrations may be accomplished by linking a processing unit with sensing device 14 and by controlling the processing unit and sensing device 14 in a manner as described below.

In the illustrated embodiment, sensing device 14 is linked with a processing unit 18 by a data link 20. Processing unit 18 may comprise, for example, a personal computer such as an Apple MAC II CPU produced by Apple Computer, Inc. However, other suitable processing units may be employed as an alternative to the Apple MAC II CPU. Data link 20 may comprise any suitable means for communicating data between sensing device 14 and processing unit 18. For example, data link 20 may be a hard wire data link, or any suitable radiation type data link, such as a radio link, a light wave link, a microwave link, or the like. The coupling, transmission, and reception devices employed with data link 20 will depend upon the type of data link employed. As these data link devices are well known in the art, further descriptions of these devices are omitted in the interest of simplifying the present disclosure.

In the illustrated embodiment, processing unit 18 receives data from sensing device 14 over data link 20. Processing unit 18 also receives meteorological data, such as wind speed and wind direction. Such meteorological data may be provided from a local weather station linked with processing unit 18, e.g., via a telephone modem (not shown) or other suitable data link. Preferably, however, a portable meteorological station 19 (such as a Campbell Scientific Portable Meteorological Station) is included with sensing device 14 to provide real-time meteorological information specific to the location of sensing device 14. In this preferred embodiment, chemical constituent and chemical concentration information (e.g., a voltage versus time chromatograph) from sensor 15 is buffered and preprocessed, for example, by a data logger or storage bank provided on board the portable meteorological station. This chemical information may be integrated with meteorological information (e.g., with well-known data integration techniques and devices) and then transmitted via data link 20 to processing unit 18. Alternatively, the chemical information may be transmitted to processing unit 18 independently from the transmission of meteorological information.

In one embodiment, sensing device 14 transmits information along data link 20 periodically, e.g., at a predetermined frequency. Such periodic data transmissions may be automatically provided by sensing device 14, e.g., by use of a clock and control circuit (not shown) connected with sensing device 14. Alternatively, processing unit 18 may be programmed, e.g., with an interrogation program 21, to send interrogation signals along data link 20 to sensing device 14. Upon receipt of an interrogation signal, sensing device 14 would respond by transmitting sensor and meteorological data along data link 20 to processing unit 18. Apparatus or software program 21 for performing interrogations and interrogation responses are well known in the art and are not discussed in further detail.

Data received from sensing device 14 by processing unit 18 may be analyzed within processing unit 18, using a conventional and well-known chemical analysis program 22, such as the E2 Chrom 200 program produced by Microsensor Technology, to determine chemical type and chemical concentration sensed by sensing device 14. This information may be used in two ways. First, it may be used in conjunction with an existing chemical dispersion modeling program 23, such as the National Oceanic and Atmospheric Administration's CAMEO program (inputted into processing unit 18 as shown in Figures 1 and 2), to determine an accurate model of the chemical dispersion (e.g., the chemical plume) in an environment. The accuracy of such a model may be substantially enhanced by employing a combination of real-time chromatographic information, and real-time meteorological information which is specific to the site of the chromatograph device.

Accuracy of the model can be further enhanced by employing specific site information (e.g., the known amount of chemicals which originally existed in the facility, or the known amount of chemicals remaining in the facility, dispersion characteristics of previously generated models, etc.) in the model determination process. To this end, processing unit 18 is connected with an input device 24, such as a keyboard. However, any suitable input device may be employed.

The chemical constituent and chemical concentration information may also be used in a second manner, to generate a group of computer models to back calculate the original chemical source at the point of release. That is, given a set of real-time meteorological conditions (e.g., wind speed, wind direction, etc.) and real-time chemical concentration and constituent conditions (e.g., chromatographic information) sensed at a particular location, calculations may be performed to determine the original amount of chemical released, and/or the specific location at which the chemical was released.

## Claims

1. Apparatus for monitoring airborne chemicals in an environment, characterized in that said apparatus comprises first sensing means (15), disposed in the environment, for sensing the concentrations of airborne chemicals in the environment and for providing signals representing such concentrations, processing means (18), operatively connected with said first sensing means (15), for providing a chemical dispersion model dependent upon said signals provided by said first sensing means (15).

2. Apparatus as claimed in claim 1, characterized in that said apparatus further comprises: second sensing means (19) for sensing meteorological data in the environment and for providing signals representing such data; wherein said processing means (18) is also operatively connected with said second sensing means (19) for providing said chemical dispersion model dependent upon said signals provided by said second sensing means (19).

3. Apparatus as claimed in claim 2, characterized in that said second sensing means (19) comprises wind speed and wind direction detecting means for detecting the wind speed and the wind direction in the environment.

4. Apparatus as claimed in claim 2, characterized in that said second sensing means (19) comprises a portable meteorological station disposed in the environment.

5. Apparatus as claimed in claim 2, characterized in that said apparatus further comprises data transmitting means (20) operatively connected with said first sensing means (15), said second sensing means (19) and said processing means (18), for transmitting data between said first and second sensing means (15,19) and said processing means (18).

6. Apparatus as claimed in claim 5, characterized in that said transmitting means (20) comprises a radio link.

7. Apparatus as claimed in claim 1, characterized in that said apparatus further comprises dispersion modeling control means (23) for controlling said processing means (18) to generate a chemical dispersion model dependent upon said signals provided by said first sensing means (15).

8. Apparatus as claimed in claim 7, characterized in that said dispersion modeling control means (23) comprises dispersion modeling software.

9. Apparatus as claimed in claim 1, characterized in that said apparatus further comprises interrogation means (21) for periodically interrogating said first sensing means (15) and for periodically transferring said signals provided by said first sensing means (15) to said processing means (18).

10. Apparatus as claimed in claim 9, characterized in that said interrogation means (21) comprises interrogation software for controlling said processing means (18) to periodically receive said signals provided by said first sensing means (15).

11. Apparatus as claimed in claim 1, characterized in that said apparatus further comprises input means (24), operatively connected with said processing means (18), for inputting data to said processing means (18), wherein said processing means (18) is operative for providing said chemical dispersion model dependent upon said data inputted with said input means (24).

12. A method of monitoring airborne chemicals in an environment, characterized in that said method comprises the steps of sensing the concentration of an airborne chemical at a location in the environment, sensing meteorological data in the environment, said meteorological data comprising at least one of the wind speed and the wind direction in the environment, and producing a computer generated chemical dispersion model dependent upon the sensed chemical concentration and the sensed meteorological data.

13. A method as claimed in claim 12, characterized in that said step of sensing the concentration comprises the steps of disposing a chromatograph (15) in the environment, and producing signals representing the concentration of an airborne chemical with said chromatograph (15).

14. A method as claimed in claim 12, characterized in that said step of sensing meteorological data comprises the steps of disposing a weather station (19) in the environment, and producing signals representing at least one of the wind speed and the wind direction in the environment with said weather station (19).

15. A method as claimed in claim 13, characterized in that said step of sensing meteorological data comprises the steps of disposing a weather station (19) in the environment, and producing signals representing at least one of the wind speed and the wind direction in the environment with said weather station (19).

16. A method as claimed in claim 15, characterized in that said step of producing a computer generated chemical dispersion model comprises the steps of transmitting said signals produced with said chromatograph (15) and said weather station (19) to a processing unit (18), and controlling said processing unit (18) to generate a chemical dispersion model dependent upon said signals transmitted to said processing unit (18).

17. A method of monitoring airborne chemicals in an environment, characterized in that said method comprises the steps of sensing the concentration of an airborne chemical at a location in the environment, and producing a computer generated model of the dispersion of the airborne chemical in the environment dependent upon the sensed chemical concentration.

18. A method as claimed in claim 17, characterized in that said step of sensing comprises the steps of disposing a chromatograph (15) at a location in the environment, and producing signals representing the real-time concentration of the airborne chemical at the location with said chromatograph (15).

19. A method as claimed in claim 18, characterized in that said method further comprises the steps of disposing a wind sensing device (19) in the environment, sensing at least one of the wind speed and the wind direction in the environment with said wind sensing device (19), and producing signals representing at least one of the wind speed and the wind direction sensed by said wind sensing device (19), wherein said step of producing a computer generated model comprises the steps of transmitting said signals produced with said chromatograph and wind sensing devices (15, 19) to a processor (18) and generating said model dependent upon said transmitted signals with said processor (18).

20. A method as claimed in claim 19, characterized in that said method further comprises the step of controlling said processor (18) with modeling software (23) to generate said model.

# FIG.1

# FIG.2

EP 0 448 360 A1

**European Patent Office**

# EUROPEAN SEARCH REPORT

EP 91 30 2380

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | ARCHIV FÜR TECHNISCHES MESSEN, no. 395, vol. 723-30, December 1968, pages 257-262; H.W. GEORGII: "Über die Einwirkung meteorologischer Parameter auf die Immissionskonzentration luftverunreinigender Komponenten" * Whole document * --- | 1-3 | G 01 N 33/00 |
| Y | ISA TRANSACTIONS, vol. 14, no. 2, 1975, pages 137-144; G.R. MARCHANT et al.: "Design philosophy of air quality surveillance systems" * Page 143, paragraph: "The predictive models" * --- | 1-3 | |
| A | MESURES, REGULATION AUTOMATISME, vol. 48, no. 6, April 1983, pages 7-21, Paris, FR; M.O. MIZIER: "Mesures de pollution" * Page 20, paragraph: "Un exemple" * --- | 1 | |
| A | ISA TRANSACTIONS, vol. 10, no. 4, 1971, pages 356-362; R. VILLALOBOS et al.: "A gas chromatographic method for automatic monitoring of pollutants in ambient air" * Whole document * --- | 1 | TECHNICAL FIELDS SEARCHED (Int. Cl.5) G 01 N |
| A | DE-A-2 518 909 (B. SAPUNOFF) * Claim 1 * ----- | 1 | |

The present search report has been drawn up for all claims

| Place of search | Date of completion of the search | Examiner |
|---|---|---|
| THE HAGUE | 12-06-1991 | DUCHATELLIER M.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

..................................................................................................................
& : member of the same patent family, corresponding document

EPO FORM 1503 03.82 (P0401)

8